# EUROPEAN PATENT SPECIFICATION

(11) **EP 4 376 911 B1**
(45) Date of publication and mention of the grant of the patent: **10.12.2025**
(21) Application number: 22751790.1
(22) Date of filing: 22.07.2022
(51) Int. Cl.: A61L 29/04, A61L 29/14

(54) **PACKAGED INTERMITTENT CATHETERS**
VERPACKTE INTERMITTIERENDE KATHETER
CATHÉTERS INTERMITTENTS EMBALLÉS

(30) Priority: 27.07.2021 US 202163203594 P
(43) Date of publication of application: 05.06.2024
(60) Divisional application: 25213429.1
(73) Proprietor: ConvaTec Limited, Deeside, Flintshire CH5 2NU (GB)
(72) Inventor: KANDRAC, Lukas, 071 01 Michalovce (SK); POLLARD, David, Deeside Flintshire CN5 2NU (GB); PYTEL, Rachel Zimet, Las Vegas, Nevada (US)
(74) Representative: Wilson Gunn
(86) International application number: PCT/GB2022/051923
(87) International publication number: WO 2023/007130

(56) References cited:
- WO-A1-2006/037321
- WO-A1-2021/092271
- US-A1- 2005 137 582
- US-A1- 2016 022 877

## Description

### Technical Field of the Invention

The present invention relates to packaged intermittent catheters and in particular those housed in a packaging container comprising an aqueous solution. The present invention also relates to methods of reducing migration of an additive from a surface of an intermittent catheter and to uses of transportation or wetting agents in aqueous solution to reduce migration of a lubricious additive from a surface of an intermittent catheter.

### Background to the Invention

Intermittent urinary catheterisation is a process involving insertion of a urinary catheter through an individual's urethra and into their bladder, where it is retained to empty the bladder of urine for only the time period that is required for emptying, after which the catheter is removed. The process differs from long-term catheterisation, which makes use of an indwelling or Foley catheter that is inserted into the bladder for long periods of time (several days to months) to discharge the residual urine of the bladder continuously throughout the day.

Intermittent catheterisation is often used by patients suffering from abnormalities of the urinary system, resulting in urinary incontinence and/or a lack of control in permitting voluntary urination. Such individuals would typically make use of intermittent catheters several times a day.

Intermittent catheters are useful devices, providing users with independence and freedom to self-catheterise as and when required, without having to rely on trained personnel to be present. This, however, increases the need for intermittent catheters to be user friendly: in particular, both easy to insert and remove with minimum discomfort caused, and safe to use with features for minimising risk of infection. Users often report experiencing pain and discomfort upon insertion and/or removal of intermittent catheters. Users have, for instance, reported experiencing bladder spasms, burning sensations, and bleeding. Urinary tract infections (UTI) are also common in individuals who practice intermittent catheterisation.

Surface coatings and additives for intermittent catheters have been used to help in alleviating these issues. However, intermittent catheter surface coatings and additives have the tendency to migrate out of the catheter with time and use, which causes the surface of the catheter to become less lubricious. Often coatings and additives may migrate out of a catheter even when the catheter is not in use and when it may be packaged in a storage or transportation medium.

In use, scraping of the catheter surface may occur, further accelerating the removal of any surface coatings or additives.

Furthermore, when a person uses an intermittent catheter, some of the coating may be left inside the user's body, which can be harmful and thus unacceptable.

To address some of the above problems, US patents US 10 058 638 B2, US 9 186 438 B2 and WO 2006/037321 A1 describe the use of a catheter containing a polymer mixture of a thermoplastic or thermo-curing polymer base material and an amphiphilic block copolymer lubricious additive. The amphiphilic block copolymer contains both a hydrophobic and hydrophilic portion. The hydrophilic portion diffuses to the surface of the catheter due to incompatibility with the hydrophobic base material and provides for a lubricious surface coating. Interactions between the hydrophobic portion of the amphiphilic molecule and the base material assist in reducing migration of the amphiphilic molecule from the catheter. Whilst this method has its advantages, there exists a need for further safeguards to reduce migration of surface coatings and additives from catheters.

There is a particular need for intermittent catheters that can be packaged in a storage or transportation medium with reduced migration of surface coatings and additives from the catheters.

It is an aim of embodiments of the present invention to address one or more of the above problems by providing an intermittent catheter, suitable for self-catheterisation use, which provides one or more of the following advantages:
- A lubricous, non-stick surface making the intermittent catheter easier to insert and remove.
- Reduced migration of coatings and additives in the intermittent catheter.
- Reduced migration of coatings and additives out of the intermittent catheter, which have the potential to enter a person's body upon use of the catheter.
- Reduced migration of coatings and additives out of the intermittent catheter when the catheter is not in use and when it may be packaged in a storage or transportation medium.

It is also an aim of embodiments of the invention to overcome or mitigate at least one problem of the prior art, whether expressly described herein or not.

### Summary of the Invention

According to a first aspect of the invention, there is provided a packaged intermittent catheter, the intermittent catheter comprising a hollow polymeric tubular body comprising a base polymer and an amphiphilic lubricious additive, housed in a packaging container; and further comprising an aqueous solution in the packaging container; wherein the aqueous solution comprises at least one salt in a concentration of at least 5% w/v.

In some embodiments, the aqueous solution comprises at least one surfactant and/or poly (alkylene oxide) compound with a total concentration of at least 0.005 mmol/L.

The amphiphilic lubricious additive comprises a hydrophobic portion and a hydrophilic portion. In cases where the base polymer is hydrophobic or generally hydrophobic, such as a polyolefin, the amphiphilic additive will diffuse towards and to an outer surface of the catheter body due to incompatibility of the hydrophilic portion of the amphiphilic additive with the hydrophobic base polymer. When the hydrophilic portion of an amphiphilic additive is present at or on the outer surface it enables wetting of the outer surface simply by applying water or a wetting agent or by wiping with a wet wipe, to create a lubricious coating, making the intermittent catheter easier and less painful to use, especially for individuals practicing self-catheterisation.

When such an aqueous solution of the invention contacts the surface of the intermittent catheter, it manipulates the energetic environment of or around the surface to reduce migration of the lubricious additive from the surface of the catheter and into the environment at and/or on the surface. This allows for the intermittent catheter to retain its lubricious surface after longer storage periods, without loss of lubricious additive into the surrounding solution.

Furthermore, the intermittent catheter retains residual aqueous solution on the catheter surface even after removal from the packaging. The residual aqueous solution provides additional lubrication to the catheter surface and may provide benefits for the user. In some embodiments, the aqueous solution mimics the salinity of urine and helps to restore osmotic balance to the urethral tissue of the user.

The aqueous solution comprises at least one salt in a concentration of at least 5% w/v.

An aqueous solution comprising a salt in such a concentration confers high polarity to the aqueous solution. When this polar solution contacts the surface of the intermittent catheter, it makes it less energetically favourable for the hydrophobic, non-polar portion of the amphiphilic additive to go into solution and migrate out of the catheter. Where the base polymer is hydrophobic or generally hydrophobic, the hydrophobic portion has an even stronger preference to interact with the base polymer compared with the external aqueous salt solution meaning that migration of the additive out of the catheter is further reduced.

In some embodiments, the aqueous solution comprises more than one salt, wherein the total concentration of the salts is at least 5% w/v.

The salt or the total amount of salts may comprise a total concentration of at least 5.5, 6, 6.5, 7, 7.5, 8, 8.5, 9, 9.5, 10, 11, 12, 13, 14, 15, 16, 17, 18, 19, 20, 21, 22, 23, 24, 25, 26, 27, 28, 29, 30, 31, 32, 33, 34, or of at least 35% w/v of the aqueous solution.

The salt or the total amount of salts may comprise a total concentration of no more than 80% w/v, or of no more than 79, 78, 77, 76, 75, 74, 73, 72, 71, 70, 69, 68, 67, 66, 65, 64, 63, 62, 61, 60, 59, 58, 57, 56 or of no more than 55% w/v; of the aqueous solution.

The salt or the total amount of salts may comprise a total concentration of between 5-50% w/v.

The salt or the total amount of salts may comprise a total concentration of between 6-50% w/v, or of between 7-50, 8-50, 9-50, 10-50, 15-50, 20-50, 25-50, 30-50, 35-50, 40-50, or of between 45-50% w/v.

The salt or the total amount of salts may comprise a total concentration of between 5-45% w/v, or of between 5-40, 5-35, 5-30, 5-25, 5-20, 5-15, 5-10, 5-9, 5-8, 5-7, or of between 5-6% w/v.

The salt or the total amount of salts may comprise a total concentration of between 6-45% w/v, or of between 6-45, 7-45, 8-45, 9-45, 10-45, 15-45, 20-45, 25-45, 30-45, 35-45, 40-45, 6-40, 7-40, 8-40, 9-40, 10-40, 15-40, 20-40, 25-40, 30-40, 35-40, 6-35, 7-35, 8-35, 9-35, 10-35, 15-35, 20-35, 25-35, 30-35. 6-30, 7-30, 8-30, 9-30, 10-30, 15-30, 20-30, 25-30, 6-25, 7-25, 8-25, 9-25, 10-25, 15-25, 20-25, 6-20, 7-20, 8-20, 9-20, 10-20, 15-20, 6-15, 7-15, 8-15, 9-15, 10-15, 6-10, 7-10, 8-10, 9-10, 6-9, 7-9, 8-9, 6-8, 7-8, or of between 6-7% w/v.

The at least one salt may comprise a cation selected from the group comprising: ammonium, calcium, iron, magnesium, potassium, pyridinium, quaternary ammonium, sodium, copper, aluminium, lithium, beryllium, strontium, and zinc. The at least one salt may comprise an anion selected from the group comprising: acetate, carbonate, bicarbonate, chloride, citrate, glutamate, fluoride, bromide, iodide, nitrate, nitrite, oxide, phosphate, ferrocyanide, silicate, gluconate, and sulfate.

The at least one salt may be selected from the group comprising: sodium chloride, potassium chloride, calcium chloride, magnesium chloride, calcium chloride, sodium nitrite, magnesium nitrate, calcium nitrate, potassium carbonate, sodium carbonate, sodium bicarbonate, potassium iodide, copper iodide, sodium ferrocyanide, monosodium glutamate, calcium silicate, sodium citrate, potassium citrate, sodium phosphate, potassium phosphate, sodium sulfate, calcium sulfate, sodium gluconate, calcium gluconate, potassium gluconate, sodium acetate, and potassium acetate.

In some embodiments, the aqueous solution comprises at least one poly (alkylene oxide) or a derivative thereof in a concentration of at least 0.005 mmol/L.

The poly (alkylene oxide) in the solution mimics the structure and/or hydrophilicity of the hydrophilic portion of the additive, which decreases the concentration gradient between the hydrophilic portions of the additive molecules and the solution. This makes it less energetically favourable for the additive to migrate out of the catheter and into solution. In addition, the poly (alkylene oxide) in the solution increases the viscosity of the solution, which in turn also makes it harder for the additive to migrate out of the catheter.

In some embodiments, the aqueous solution comprises more than one poly (alkylene oxide) or a derivative thereof, wherein the total concentration of the poly (alkylene oxide) compounds or derivatives is at least 0.005 mmol/L.

The poly (alkylene oxide) or derivative thereof or the total amount of poly (alkylene oxide) compounds or derivatives may comprise a concentration of at least 0.0006, 0.0007, 0.0008, 0.0009, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or of at least 300 mmol/L.

The poly (alkylene oxide) or derivative thereof or the total amount of poly (alkylene oxide) compounds or derivatives may comprise a concentration of no more than 1000 mmol/L, or of no more than 995, 990, 985, 980, 975, 970, 965, 960, 955, 950, 945, 940, 935, 930, 925, 920, 915, 910, 905, 900, 895, 890, 885, 880, 875, 870, 865, 860, 855, 850, 845, 840, 835, 830, 825, 820, 815, 810, 805, 800, 795, 790, 785, 780, 775, 770, 765, 760, 755, 750, 745, 740, 735, 730, 725, 720, 715, 710, 705 or of no more than 700 mmol/L.

The poly (alkylene oxide) or derivative thereof or the total amount of poly (alkylene oxide) compounds or derivatives may comprise a concentration of between 0.005-500 mmol/L.

The poly (alkylene oxide) or derivative thereof or the total amount of poly (alkylene oxide) compounds or derivatives may comprise a concentration of between 0.01-500 mmol/L, or of between 0.05-500, 0.1-500, 0.5-500, 1-500, 5-500, 10-500, 50-500, 100-500, 150-500, 200-500, 250-500, 300-500, 350-500, 400-500, or of between 450-500 mmol/L.

The poly (alkylene oxide) or derivative thereof or the total amount of poly (alkylene oxide) compounds or derivatives may comprise a concentration of between 0.005-450 mmol/L, or of between 0.005-400, 0.005-350, 0.005-300, 0.005-250, 0.005-200, 0.005-150, 0.005-100, 0.005-50, 0.005-10, 0.005-5, 0.005-1, 0.005-0.5, 0.005-0.1, 0.005-0.05, or of between 0.005-0.01 mmol/L.

The poly (alkylene oxide) or derivative thereof or the total amount of poly (alkylene oxide) compounds or derivatives may comprise a concentration of between 0.01-450 mmol/L, or ofbetween 0.05-450, 0.1-450, 0.5-450, 1-450, 5-450, 10-450, 50-450, 100-450, 150-450, 200-450, 250-450, 300-450, 350-450, 400-450, 0.01-400, 0.05-400, 0.1-400, 0.5-400, 1-400, 5-400, 10-400, 50-400, 100-400, 150-400, 200-400, 250-400, 300-400, 350-400, 0.01-350, 0.05-350, 0.1-350, 0.5-350, 1-350, 5-350, 10-350, 50-350, 100-350, 150-350, 200-350, 250-350, 300-350, 0.01-300, 0.05-300, 0.1-300, 0.5-300, 1-300, 5-300, 10-300, 50-300, 100-300, 150-300, 200-300, 250-300, 0.01-250, 0.05-250, 0.1-250, 0.5-250, 1-250, 5-250, 10-250, 50-250, 100-250, 150-250, 200-250, 0.01-200, 0.05-200, 0.1-200, 0.5-200, 1-200, 5-200, 10-200, 50-200, 100-200, 150-200, 0.01-150, 0.05-150, 0.1-150, 0.5-150, 1-150, 5-150, 10-150, 50-150, 100-150, 0.01-100, 0.05-100, 0.1-100, 0.5-100, 1-100, 5-100, 10-100, 50-100, 0.01-50, 0.05-50, 0.1-50, 0.5-50, 1-50, 5-50, 10-50, 0.01-10, 0.05-10, 0.1-10, 0.5-10, 1-10, 5-10, 0.01-5, 0.05-5, 0.1-5, 0.5-5, 1-5, 0.01-1, 0.05-1, 0.1-1, 0.5-1, 0.01-0.5, 0.05-0.5, 0.1-0.5, 0.01-0.1, 0.05-0.1, or of between 0.01-0.05 mmol/L.

In some embodiments, the at least one poly (alkylene oxide) is selected from the group comprising: polyethylene oxide and polypropylene oxide.

In some embodiments, the at least one poly (alkylene oxide) comprises polyethylene oxide with an average molecular weight of at least 100 g/mol, or of at least 200, 300, 400, 500, 600, 700, 800, 900, or of at least 1000 g/mol.

In some embodiments, the at least one poly (alkylene oxide) comprises polyethylene oxide with an average molecular weight of no more than 10000000 g/mol, or of no more than 9000000, 8000000, 7000000, 6000000, 5000000, 4000000, 3000000, 2000000, 1000000, 900000, 800000, 700000, 600000, 500000, 400000, 300000, 200000, 100000, 90000, 80000, 70000, 60000, 50000, 40000, 30000, 20000, or of no more than 10000 g/mol.

In some embodiments, the aqueous solution comprises at least one surfactant in a concentration of at least 0.005 mmol/L.

In some embodiments, the aqueous solution comprises more than one surfactant, wherein the total surfactant concentration is at least 0.005 mmol/L.

Statements above relating to the concentration of poly (alkylene oxide) or derivatives thereof may also be applied to the total concentration of the surfactant or the total amount of surfactants.

In some embodiments, the at least one surfactant comprises an amphiphilic molecule comprising a hydrophilic-lipophilic balance (HLB) of at least 5, or of at least 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, or of at least 18.

In some embodiments, the amphiphilic molecule comprises an HLB of between 7-20.

In some embodiments, the amphiphilic molecule comprises an HLB of between 8-20, 9-20, 10-20, 11-20, 12-20, 13-20, 14-20, 15-20, 16-20, 17-20, 18-20, or of between 19-20.

In some embodiments, the amphiphilic molecule comprises an HLB of between 7-19, 7-18, 7-17, 7-16, 7-15, 7-14, 7-13, 7-12, 7-11, 7-10, 7-9, or of between 7-8.

In further embodiments, the amphiphilic molecule comprises an HLB of between 8-19, 9-19, 10-19, 11-19, 12-19, 13-19, 14-19, 15-19, 16-19, 17-19, 18-19, 8-18, 9-18, 10-18, 11-18, 12-18, 13-18, 14-18, 15-18, 16-18, 17-18, 8-17, 9-17, 10-17, 11-17, 12-17, 13-17, 14-17, 15-17, 16-17, 8-16, 9-16, 10-16, 11-16, 12-16, 13-16, 14-16, 15-16, 8-15, 9-15, 10-15, 11-15, 12-15, 13-15, 14-15, 8-14, 9-14, 10-14, 11-14, 12-14, 13-14, 8-13, 9-13, 10-13, 11-13, 12-13, 8-12, 9-12, 10-12, 11-12, 8-11, 9-11, 10-11, 8-10, 9-10, or of between 8-9.

The HLB value of the amphiphilic lubricious additive is calculated using Griffin's method, wherein ***HLB = 20** ** ***Mₕ*/*_{M}* (*Mₕ*** is the molecular mass of the hydrophilic portion of the amphiphilic molecule, and ***M*** is the molecular mass of the whole molecule).

The surfactant may be ionic or non-ionic. The ionic surfactant may comprise a functional group selected from the group comprising: sulfate, sulfonate, phosphate, carboxylate, and ammonium. The ionic surfactant may be selected from the group comprising: sodium lauryl sulfate, sodium laureth sulfate, ammonium lauryl sulfate, ammonium laureth sulfate, sodium stearate, potassium cocoate, sodium lauryl sarcosinate, sodium myreth sulfate, sodium pareth sulfate, sodium dodecylbenzenesulfonate, cetyltrimethylammonium bromide, potassium cetyl phosphate, sodium stearoyl glutamate, and glyceryl stearate citrate.

The non-ionic surfactant may be selected from the group comprising: alkyl polyglucosides, such as decyl glucoside, lauryl glucoside and octyl glucoside; fatty acid esters of polyhydroxy compounds, which may comprise fatty acid esters of glycerol (such as glycerol monostearate and glycerol monolaurate), fatty acid esters of sorbitol (such as Spans, such as sorbitan monolaurate, sorbitan monostearate and sorbitan tristearate, and Tweens, such as Tween 20, Tween 40, Tween 60, and Tween 80), and fatty acid esters of sucrose; and ethoxylates, which may comprise fatty alcohol ethoxylates (such as narrow-range ethoxylates, octaethylene glycol monododecyl ether, and pentaethylene glycol monododecyl ether), fatty acid ethoxylates, ethoxylated fatty esters and oils, ethoxylated amines and/or fatty acid amides (such as polyethoxylated tallow amine, cocamide monoethanolamine, and cocamide diethanolamine), terminally blocked ethoxylates (such as poloxamers), and alkylphenol ethoxylates (such as nonoxynols and Triton-X).

In some embodiments, the aqueous solution comprises at least one surfactant and at least one poly (alkylene oxide) compound with a combined surfactant and poly (alkylene oxide) concentration of at least 0.005 mmol/L.

Statements above relating to the concentration of poly (alkylene oxide) or derivatives thereof may also be applied to the combined surfactant and poly (alkylene oxide) concentration.

In some embodiments, the intermittent catheter amphiphilic lubricious additive is polymeric or oligomeric. At least some of the additive may be at or on the outer surface of the body. By "at the outer surface", it is meant that at least a portion of the additive forms part of the surface of protrudes from the surface.

The outer surface may comprise at least one member of the group comprising: the external-facing surface of the body, the lumen of the body and any eyelets present on the body. In preferred embodiments the outer surface is the external-facing surface of the body and/or the inner lumen. In some embodiments, the outer surface may comprise the external-facing surface of the body of the catheter, the inner lumen, and the eyelets.

The hydrophilic portion of at least some of the additive may protrude from or form part of the outer surface of the body, while at least part of the hydrophobic portion may be retained or anchored within the polymer body.

The additive may be concentrated at or on the outer surface of the body. For example, at least 25%, 30%, 35%, 40%, 45%, 50%, 55%, 60%, 65%, 70%, 75% or at least 80% of the number of molecules of the additive may be at or on the outer surface of the body.

In some embodiments, at least 25%, 30%, 35%, 40%, 45%, 50%, 55%, 60%, 65%, 70%, 75% or at least 80% of the number of molecules of additive may have hydrophilic portions that are at or on the outer surface of the body.

In some embodiments, the additive is located at and/or on at least 50, 55, 60, 65, 70, 75, 80, 85, 90, 95, 96, 97, 98 or at least 99% of the outer surface area of the polymeric tubular body, preferably at least 75% or at least 90% of the outer surface area of the polymeric tubular body or between 75% and 100% of the outer surface area.

In some embodiments, the additive comprises a concentration of at least 0.1, 0.2, 0.3. 0.4. 0.5, 0.75, 1, 2, 3, 4, 5, 10, 15 or at least 20%, preferably between 0.1-20%, and more preferably between 0.5-15% or 0.5-5% by weight of the combination of base polymer and additive.

In some embodiments, the additive is an A-B block copolymer comprising a hydrophobic hydrocarbon A-block and a hydrophilic B-block. The hydrophobic portion (A-block) may comprise a carbon chain of at least 5 carbon atoms, or at least 10, 15, 20, 25, 30, 35, or 40 carbon atoms. The hydrophobic portion may preferably comprise a carbon chain of between 20-52 carbon atoms.

In some embodiments, the A-block comprises a hydrocarbon chain block of the formula CH₃CH₂(CH₂CH₂)ₐ. The value of "a" may be between 5-25; for instance, "a" may be 5, 6, 7, 8, 9, 10, 11, 12, 13, 14, 15, 16, 17, 18, 19, 20, 21, 22, 23, 24, or 25, or a half integer of any of the above values. The value of "a" may preferably be between 9-25; for instance, "a" may be 9, 10, 11, 12, 13, 14, 15, 16, 17, 18, 19, 20, 21, 22, 23, 24, or 25, or a half integer of any of the above values.

In some embodiments, the B-block is a hydrophilic oligomer, i.e. a homo- or co-oligomer, comprising between 2 and 10 monomer units. The monomer units may be selected from the group comprising: alkylene oxides, alkylene glycols, epihalohydrins, unsaturated carboxylic acids, alkylene imines, lactones, vinyl alcohol, and vinyl alkanoates. The monomer units may be preferably selected from the group comprising: ethylene oxide, propylene oxide, ethylene glycol, propylene glycol, epichlorohydrin, acrylic acid, methacrylic acid, ethylene imine, caprolactone, vinyl alcohol, and vinyl acetate. In some embodiments the monomer units comprise alkylene oxide groups independently selected from ethylene oxide and propylene oxide, and in preferred embodiments all of the monomer units are ethylene oxide or all of the monomer units are propylene oxide.

In preferred embodiments, the intermittent catheter base polymer is hydrophobic or partly hydrophobic. A hydrophobic base polymer facilitates increased hydrophobic-hydrophobic interactions between the hydrophobic portion of the additive and the base polymer. This further decreases the energetic favourability for the hydrophobic portion to leave the base polymer and migrate out into the more hydrophilic external environment.

In some embodiments, the base polymer comprises a polymer selected from the group comprising: polyvinyl chloride, polytetrafluoroethylene, polyolefins, latex, silicones, synthetic rubbers, polyurethanes, polyesters, polyacrylates, polyamides, thermoplastic elastomeric materials, styrene block copolymers, polyether block amide, thermoplastic vulcanizates, thermoplastic copolyesters, thermoplastic polyamides, styrene-butadiene copolymer (SBC), styrene-ethylene-butylene-styrene copolymer (SEBS), and water disintegrable or enzymatically hydrolysable material, or combinations, blends or copolymers of any of the above materials.

In preferred embodiments, the base polymer comprises a polymer selected from the group comprising: polyolefins, polyesters, polyacrylates, polyamides, thermoplastic elastomeric material, polyether block amide, thermoplastic vulcanizates, thermoplastic copolyesters, thermoplastic polyamides, fluororubber, and water disintegrable or enzymatically hydrolysable material or combinations, blends or copolymers of any of the above materials.

In some embodiments, said water disintegrable or enzymatically hydrolysable material comprises a material of the group comprising: polyvinyl alcohol, extrudable polyvinyl alcohol, polyacrylic acids, polylactic acid, polyesters, polyglycolide, polyglycolic acid, poly lactic-co-glycolic acid, polylactide, amines, polyacrylamides, poly(N-(2-Hydroxypropyl) methacrylamide), starch, modified starches or derivatives, amylopectin, pectin, xanthan, scleroglucan, dextrin, chitosans, chitins, agar, alginate, carrageenans, laminarin, saccharides, polysaccharides, sucrose, polyethylene oxide, polypropylene oxide, acrylics, polyacrylic acid blends, poly(methacrylic acid), polystyrene sulfonate, polyethylene sulfonate, lignin sulfonate, polymethacrylamides, copolymers of aminoalkyl-acrylamides and methacrylamides, melamine-formaldehyde copolymers, vinyl alcohol copolymers, cellulose ethers, poly-ethers, polyethylene oxide, blends of polyethylene- polypropylene glycol, carboxymethyl cellulose, guar gum, locust bean gum, hydroxypropyl cellulose, vinylpyrrolidone polymers and copolymers, polyvinyl pyrrolidone-ethylene-vinyl acetate, polyvinyl pyrrolidone-carboxymethyl cellulose, carboxymethyl cellulose shellac, copolymers of vinylpyrrolidone with vinyl acetate, hydroxyethyl cellulose, gelatin, poly-caprolactone, poly(p-dioxanone), or combinations, blends or co-polymers of any of the above materials.

In other preferred embodiments, the base polymer comprises a polymer selected from the group comprising: polyolefins, polyvinyl chloride, polyurethane, styrene-butadiene copolymer (SBC), styrene-ethylene-butylene-styrene copolymer (SEBS), and thermoplastic elastomeric material or combinations, blends or copolymers of any of the above materials.

In some preferred embodiments, the base polymer comprises a polyolefin, especially polyethylene and/or polypropylene.

In some preferred embodiments, the base polymer comprises a thermoplastic elastomeric material. The base polymer may comprise a thermoplastic polyolefin.

The thermoplastic base polymer may comprise a hydrophobic polymer selected from the group comprising: Accurel ^{™}, Styroflex^{™}, Styrolux^{™}, MelifleX^{™}, and Mediprene^{™}.

The thermoplastic base polymer may comprise Estane^{™} 58315, which is both hydrophobic and hydrophilic.

In some embodiments, an outer surface of the polymeric tubular body comprises a separate or further lubricating agent or bacteria-repellent agent at and/or on the surface, in addition to the additive. The separate or further lubricating agent or bacteria-repellent agent may be bonded at and/or on the surface.

In some embodiments, said further lubricating agent or bacteria-repellent agent is formed from a coating material selected from the group comprising: silver-based, polytetrafluoroethylene, hydrogel, silicone, lecithin, salicylic acid, minocycline, rifampin, fluorinated ethylene propylene, polyvinylidone, polyvinyl compounds, polylactames, polyvinyl pyrrolidones, polysaccharides, heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, polyethylene oxides, polyvinyl alcohols, polyamides, polyacrylic acid, hydroxy ethylmethyl acrylate, polymethylvinyl ether, maleinic acid anyhydride, penicillin, neomycin sulfate, cephalothin, Bacitracin, phenoxymethyl penicillin, lincoymycin hydrochloride, sulfadiazine, methyl sulfadiazine, succinoylsulfathiazole, phthalylsulfathiazde, sulfacetamine, procaine penicillin, streptomycin, aureomycin, terramycin, terramycin, quaternary ammonium halides, cetyl pyridinium chloride, triethyl dodecyl ammonium bromide, hexachlorophene and nitrofurazone, or any combination thereof.

In some embodiments, the aqueous solution is in direct contact with the intermittent catheter, and preferably with at least one surface thereof. The at least one surface may comprise the outer surface of the intermittent catheter. The solution may cover at least part of the outer surface of the catheter.

The at least one surface may comprise an inner surface of the catheter and the solution may cover at least part of the inner surface.

In some embodiments, the intermittent catheter is submerged in the aqueous solution.

In some embodiments, the packaging container may comprise a storage or transportation container and the aqueous solution may comprise a storage or transportation medium. The solution may be in direct contact with the intermittent catheter allowing for reduced migration of the lubricious additive out of the catheter during storage or transportation.

The aqueous solution may act as a wetting agent. The solution may be present on the outer surface of the catheter and may both reduce additive migration out of the catheter and encourage the hydrophilic portions of the amphiphilic additive molecules to seek towards the outer surface of the catheter due to their affinity with the hydrophilic aqueous solution. Increased numbers of hydrophilic portions of additive molecules at or on the outer surface increase the lubricity of the surface, which makes the catheter easier and less painful to insert and remove.

In some embodiments, the aqueous solution is contained within a separate container, such as a bag or sachet, within the container housing and is not in direct contact with the intermittent catheter.

The separate container may be pierceable, in use, to release the contained aqueous solution from the separate container and into direct contact with the intermittent catheter.

Prior to inserting the intermittent catheter, the user may release the aqueous solution from the separate container and apply the solution to the outer surface of the catheter. The solution may assist in both reducing migration of the additive out of the catheter and in increasing the lubricity of the catheter surface. Reducing migration of the additive out of the catheter is particularly important when the catheter is in use, as leaching of the additive into the user's body can be harmful.

According to a second aspect of the invention, there is provided a method of reducing migration of an additive from a surface of an intermittent catheter, the intermittent catheter comprising a hollow polymeric tubular body comprising a base polymer and an amphiphilic lubricious additive, the method comprising the step of packaging the intermittent catheter in a container comprising an aqueous solution, wherein at least part of the intermittent catheter is covered by the aqueous solution, and wherein the aqueous solution comprises at least one salt in a concentration of at least 5% w/v.

The aqueous solution may comprise any aqueous solution defined for the first aspect of the invention. Statements of invention relating to the aqueous solution of the first aspect of the invention may also be applied to the second aspect of the invention.

The intermittent catheter may comprise any intermittent catheter of the first aspect of the invention. Statements of invention relating to the intermittent catheter of the first aspect of the invention or to any of its components may also be applied to the second aspect of the invention.

The at least part of the intermittent catheter that is covered by the aqueous solution may comprise part of or the entirety of at least one surface of the intermittent catheter. The at least one surface may comprise an outer and/or inner surface of the catheter, and preferably an outer surface.

In some embodiments, the method comprises submerging the intermittent catheter in the aqueous solution.

In some embodiments, the packaging container may comprise a storage or transportation container and the aqueous solution may comprise a storage or transportation medium. Covering at least part of the intermittent catheter with the aqueous solution can reduce migration of the lubricious additive out of the catheter during storage or transportation.

The aqueous solution may act as a wetting agent. Covering at least part of the outer surface of the intermittent catheter with the aqueous solution may both reduce additive migration out of the catheter and encourage the hydrophilic portions of the amphiphilic additive molecules to seek towards the outer surface of the catheter due to their affinity with the hydrophilic aqueous solution. Increased numbers of hydrophilic portions of additive molecules at or on the outer surface increase the lubricity of the surface, which makes the catheter easier and less painful to insert and remove.

According to a third aspect of the invention, there is provided the use of an aqueous solution comprising at least one salt in a concentration of at least 5% w/v, to reduce migration of a lubricious additive from a surface of an intermittent catheter, the intermittent catheter comprising a hollow polymeric tubular body comprising a base polymer and an amphiphilic lubricious additive.

The aqueous solution may comprise any aqueous solution defined for the first aspect of the invention. Statements of invention relating to the aqueous solution of the first aspect of the invention may also be applied to the third aspect of the invention.

The intermittent catheter may comprise any intermittent catheter of the first aspect of the invention. Statements of invention relating to the intermittent catheter of the first aspect of the invention or to any of its components may also be applied to the third aspect of the invention.

The aqueous solution may be applied to the surface of the intermittent catheter to reduce migration of the lubricious additive from the surface.

The intermittent catheter may be submerged in the aqueous solution to reduce migration of the lubricious additive from the surface of the intermittent catheter.

The surface of the intermittent catheter may comprise an outer surface or an inner surface of the catheter, and preferably an outer surface.

The aqueous solution may be used to reduce migration of the lubricious additive from the surface of the intermittent catheter during transportation of the catheter.

The aqueous solution may be used to reduce migration of the lubricious additive from the surface of the intermittent catheter during use of the catheter. The aqueous solution may be used to both reduce additive migration out of the catheter and encourage hydrophilic portions of the amphiphilic additive molecules to seek towards the outer surface of the catheter due to their affinity with the hydrophilic aqueous solution. Increased numbers of hydrophilic portions of additive molecules at or on the outer surface increase the lubricity of the surface, which makes the catheter easier and less painful to insert and remove.

### Detailed Description of the Invention

In order that the invention may be more clearly understood embodiments thereof will now be described, by way of example only:

### Example 1:

A first embodiment of a packaged intermittent catheter of the invention comprises an intermittent catheter comprising a hollow polymeric tubular body comprising a base polymer formed of thermoplastic polypropylene and further comprising an amphiphilic additive of the formula CH₃CH₂(CH₂CH₂)₁₅(OCH₂CH₂)₅OH. The amphiphilic additive comprises a hydrophilic block which seeks towards the outer surface of the body due to its incompatibility with the base polymer, the outer surface becoming lubricious as a result. The lipophilic and hydrophobic block of the amphiphilic additive ensures that the hydrophilic block is secured to the base material.

The intermittent catheter may be prepared as described in US patents US 10 058 638 B2 and US 9 186 438 B2.

The intermittent catheter is housed in a packaging container which also comprises an aqueous solution comprising 10% w/v of sodium chloride salt. The intermittent catheter is submerged in the aqueous solution.

The intermittent catheter is used in the conventional manner upon removal from the container.

The aqueous solution comprising sodium chloride salt in such a concentration confers high polarity to the aqueous solution. When this polar solution contacts the surface of the intermittent catheter, it makes it less energetically favourable for the hydrophobic, non-polar portion of the amphiphilic additive to go into solution and migrate out of the catheter. Due to the hydrophobic nature of the thermoplastic polypropylene base polymer, the hydrophobic portion of the additive has a strong preference to interact with the base polymer compared with the external aqueous salt solution meaning that migration of the additive out of the catheter is further reduced.

Along with reducing additive migration out of the catheter, the aqueous solution also encourages the hydrophilic portions of the amphiphilic additive molecules to seek towards the outer surface of the catheter due to their affinity with the hydrophilic aqueous solution. Increased numbers of hydrophilic portions of additive molecules at or on the outer surface increase the lubricity of the surface, which makes the catheter easier and less painful to insert and remove. No further wetting of the catheter outer surface is required to generate a lubricious surface prior to use.

The intermittent catheter of Example 1 conferred reduced migration of the amphiphilic additive from the surface of the catheter during both storage/transport and through use of the catheter compared to a control packaged intermittent catheter in which water bathed the catheter. It also provided reduced resistance to abrasion of the additive from the surface of the catheter on contact with external bodies.

### Example 2:

A second embodiment of a packaged intermittent catheter of the invention comprises an intermittent catheter comprising a hollow polymeric tubular body comprising a base polymer formed of thermoplastic polyethylene and further comprising an amphiphilic additive of the formula CH₃CH₂(CH₂CH₂)₂₀(OCH₂CH₂)₈OH. The amphiphilic additive comprises a hydrophilic block which seeks towards the outer surface of the body due to its incompatibility with the base polymer, the outer surface becoming lubricious as a result. The lipophilic and hydrophobic block of the amphiphilic additive ensures that the hydrophilic block is secured to the base material.

The intermittent catheter may be prepared as described in US patents US 10 058 638 B2 and US 9 186 438 B2.

The intermittent catheter is housed in a packaging container which also comprises an aqueous solution comprising 3% w/v of sodium chloride salt and 3% w/v of trisodium citrate salt. The aqueous solution is contained within a sachet within the container housing and is not in direct contact with the catheter. The sachet is pierceable to release the contained aqueous solution.

Prior to inserting the intermittent catheter, the user releases the aqueous solution from the sachet and applies the solution to the outer surface of the catheter. No further wetting of the catheter outer surface is required prior to use.

The aqueous solution comprising sodium chloride and trisodium citrate salts in such concentrations confers high polarity to the aqueous solution. The mechanism of action of the aqueous solution is identical to that of Example 1. When the aqueous solution had been applied to its outer surface, the intermittent catheter of Example 2 conferred reduced migration of the amphiphilic additive from the surface of the catheter during both use and subsequent storage of the catheter. It also provided reduced resistance to abrasion of the additive from the surface of the catheter on contact with external bodies.

## Claims

1. A packaged intermittent catheter, the intermittent catheter comprising a hollow polymeric tubular body comprising a base polymer and an amphiphilic lubricious additive, housed in a packaging container; and further comprising an aqueous solution in the packaging container; wherein the aqueous solution comprises at least one salt in a concentration of at least 5% w/v.

2. A packaged intermittent catheter as claimed in claim 1, wherein the aqueous solution comprises at least one surfactant and/or poly (alkylene oxide) compound with a total concentration of at least 0.005 mmol/L.

3. A packaged intermittent catheter as claimed in any preceding claim, wherein the amphiphilic lubricious additive is polymeric or oligomeric.

4. A packaged intermittent catheter as claimed in any preceding claim, wherein the amphiphilic additive is an amphiphilic A-B block copolymer comprising a hydrophobic hydrocarbon A-block and a hydrophilic B-block.

5. A packaged intermittent catheter as claimed in claim 4, wherein the amphiphilic additive is an A-B block copolymer comprising an A-block comprising a hydrocarbon chain block of the formula CH₃CH₂(CH₂CH₂)ₐ where "a" is 5-25 and preferably 9-25, and a hydrophilic B-block.

6. A packaged intermittent catheter as claimed in claim 4 or 5, wherein the B-block is a hydrophilic oligomer comprising between 2 and 10 monomer units optionally derived from monomers selected from the group consisting of: alkylene oxides, alkylene glycols, epihalohydrins, unsaturated carboxylic acids, alkylene imines, lactones, vinyl alcohol, and vinyl alkanoates.

7. A packaged intermittent catheter as claimed in any preceding claim, wherein the base polymer comprises a polymer selected from the group consisting of: polyolefins, polyesters, polyacrylates, polyamides, thermoplastic elastomeric material, polyether block amide, thermoplastic vulcanizates, thermoplastic copolyesters, thermoplastic polyamides, fluororubber, and water disintegrable or enzymatically hydrolysable material or combinations, blends or copolymers of any of the above materials.

8. A packaged intermittent catheter as claimed in claim 7, wherein the base polymer comprises a polymer selected from the group consisting of:
polyolefins, polyvinyl chloride, polyurethane, styrene-butadiene copolymer (SBC), styrene-ethylene-butylene-styrene copolymer (SEBS), and
thermoplastic elastomeric material or combinations, blends or copolymers of any of the above materials.

9. A packaged intermittent catheter as claimed in any preceding claim, wherein the amphiphilic additive is an A-B block copolymer comprising an A-block comprising a hydrocarbon chain block of the formula CH₃CH₂(CH₂CH₂)ₐ, where "a" is 5-25 and preferably 9-25, and a hydrophilic B-block, wherein the B-block is a hydrophilic oligomer comprising between 2 and 10 monomer units derived from monomers selected from the group consisting of: alkylene oxides, alkylene glycols, epihalohydrins, unsaturated carboxylic acids, alkylene imines, lactones, vinyl alcohol, and vinyl alkanoates.

10. A method of reducing migration of an additive from a surface of an intermittent catheter, the intermittent catheter comprising a hollow polymeric tubular body comprising a base polymer and an amphiphilic lubricious additive, the method comprising the step of packaging the intermittent catheter in a container comprising an aqueous solution, wherein at least part of the intermittent catheter is covered by the aqueous solution, and wherein the aqueous solution comprises at least one salt in a concentration of at least 5% w/v.

11. A method of reducing migration as claimed in claim 10, wherein the aqueous solution comprises at least one surfactant and/or poly (alkylene oxide) compound with a combined concentration of at least 0.005 mmol/L.

12. Use of an aqueous solution comprising at least one salt in a concentration of at least 5% w/v; to reduce migration of a lubricious additive from a surface of an intermittent catheter, the intermittent catheter comprising a hollow polymeric tubular body comprising a base polymer and an amphiphilic lubricious additive.

## Patentansprüche

1. Verpackter intermittierender Katheter, wobei der intermittierende Katheter einen hohlen polymeren röhrenförmigen Körper umfassend ein Basispolymer und ein amphiphiles gleitfähiges Additiv, untergebracht in einem Verpackungsbehälter, umfasst; und ferner eine wässrige Lösung in dem Verpackungsbehälter umfasst; wobei die wässrige Lösung mindestens ein Salz in einer Konzentration von mindestens 5% w/v umfasst.

2. Verpackter intermittierender Katheter nach Anspruch 1, wobei die wässrige Lösung mindestens ein Tensid und/oder eine Poly(alkylenoxid)-Verbindung mit einer Gesamtkonzentration von mindestens 0,005 mmol/L umfasst.

3. Verpackter intermittierender Katheter nach einem der vorhergehenden Ansprüche, wobei das amphiphile gleitfähige Additiv polymer oder oligomer ist.

4. Verpackter intermittierender Katheter nach einem der vorhergehenden Ansprüche, wobei das amphiphile Additiv ein amphiphiles A-B-Blockcopolymer umfassend einen hydrophoben Kohlenwasserstoff-A-Block und einen hydrophilen B-Block ist.

5. Verpackter intermittierender Katheter nach Anspruch 4, wobei das amphiphile Additiv ein A-B-Block-Copolymer umfassend einen A-Block umfassend einen Kohlenwasserstoffkettenblock der Formel CH₃CH₂(CH₂CH₂)ₐ, wobei "a" 5-25 und bevorzugt 9-25 ist, und einen hydrophilen B-Block ist.

6. Verpackter intermittierender Katheter nach Anspruch 4 oder 5, wobei der B-Block ein hydrophiles Oligomer umfassend zwischen 2 und 10 Monomereinheiten optional abgeleitet von Monomeren, ausgewählt aus der Gruppe bestehend aus: Alkylenoxiden, Alkylenglykolen, Epihalohydrinen, ungesättigten Carbonsäuren, Alkyleniminen, Lactonen, Vinylalkohol und Vinylalkanoaten, ist.

7. Verpackter intermittierender Katheter nach einem der vorhergehenden Ansprüche, wobei das Basispolymer ein Polymer ausgewählt aus der Gruppe bestehend aus: Polyolefinen, Polyestern, Polyacrylaten, Polyamiden, thermoplastischem elastomerem Material, Polyetherblockamid, thermoplastischen Vulkanisaten, thermoplastischen Copolyestern, thermoplastischen Polyamiden, Fluorkautschuk und wasserdesinfizierbarem oder enzymatisch hydrolysierbarem Material oder Kombinationen, Mischungen oder Copolymeren der oben genannten Materialien umfasst.

8. Verpackter intermittierender Katheter nach Anspruch 7, wobei das Basispolymer ein Polymer ausgewählt aus der Gruppe bestehend aus: Polyolefinen, Polyvinylchlorid, Polyurethan, Styrol-Butadien-Copolymer (SBC), Styrol-Ethylen-Butylen-Styrol-Copolymer (SEBS) und thermoplastischem elastomerem Material oder Kombinationen, Mischungen oder Copolymeren der oben genannten Materialien umfasst.

9. Verpackter intermittierender Katheter nach einem der vorhergehenden Ansprüche, wobei das amphiphile Additiv ein A-B-Block-Copolymer umfassend einen A-Block umfassend einen Kohlenwasserstoffkettenblock der Formel CH₃CH₂(CH₂CH₂)ₐ, wobei "a" 5-25 und bevorzugt 9-25 ist, und einen hydrophilen B-Block ist, wobei der B-Block ein hydrophiles Oligomer umfassend zwischen 2 und 10 Monomereinheiten abgeleitet von Monomeren ausgewählt aus der Gruppe bestehend aus: Alkylenoxiden, Alkylenglykolen, Epihalohydrinen, ungesättigten Carbonsäuren, Alkyleniminen, Lactonen, Vinylalkoholen und Vinylalkanoaten, ist.

10. Verfahren zur Verringerung der Migration eines Additivs von einer Oberfläche eines intermittierenden Katheters, wobei der intermittierende Katheter einen hohlen polymeren röhrenförmigen Körper umfassend ein Basispolymer und ein amphiphiles gleitfähiges Additiv umfasst, wobei das Verfahren den Schritt des Verpackens des intermittierenden Katheters in einem eine wässrige Lösung umfassenden Behälter umfasst, wobei mindestens ein Teil des intermittierenden Katheters von der wässrigen Lösung bedeckt ist, und wobei die wässrige Lösung mindestens ein Salz in einer Konzentration von mindestens 5 % w/v umfasst.

11. Verfahren zur Verringerung der Migration nach Anspruch 10, wobei die wässrige Lösung mindestens ein Tensid und/oder eine Poly(alkylenoxid)-Verbindung mit einer kombinierten Konzentration von mindestens 0,005 mmol/L umfasst.

12. Verwendung einer wässrigen Lösung umfassend mindestens ein Salz in einer Konzentration von mindestens 5% (w/v), um die Migration eines gleitfähigen Additivs von einer Oberfläche eines intermittierenden Katheters zu verringern, wobei der intermittierende Katheter einen hohlen polymeren röhrenförmigen Körper umfassend ein Basispolymer und ein amphiphiles gleitfähiges Additiv umfasst.

## Revendications

1. Un cathéter intermittent emballé, le cathéter intermittent comprenant un corps tubulaire creux en polymère comprenant un polymère de base et un additif lubrifiant amphiphile, logés dans un récipient d'emballage ; et comprenant en outre une solution aqueuse dans le récipient d'emballage ; dans lequel la solution aqueuse comprend au moins un sel en une concentration d'au moins 5 % en poids/volume.

2. Un cathéter intermittent emballé tel que revendiqué à la revendication 1, dans lequel la solution aqueuse comprend au moins un agent tensio-actif et/ou un composé en poly(oxyde d'alcoylène) en une concentration totale d'au moins 0,005 mmol/L.

3. Un cathéter intermittent emballé tel que revendiqué dans n'importe quelle revendication précédente, dans lequel l'additif lubrifiant amphiphile est un polymère ou un oligomère.

4. Un cathéter intermittent emballé tel que revendiqué dans n'importe quelle revendication précédente, dans lequel l'additif amphiphile est un copolymère amphiphile séquencé A-B comprenant une séquence A hydrocarbonée hydrophobe et une séquence B hydrophile.

5. Un cathéter intermittent emballé tel que revendiqué dans la revendication 4, dans lequel l'additif amphiphile est un copolymère séquencé A-B comprenant une séquence A comprenant une séquence de chaîne hydrocarbonée de la formule CH₃CH₂ (CH₂CH₂)ₐ, dans laquelle "a" va de 5 à 25 et de préférence de 9 à 25 et une séquence B hydrophile.

6. Un cathéter intermittent emballé tel que revendiqué dans la revendication 4 ou 5, dans lequel la séquence B est un oligomère hydrophile comprenant entre 2 et 10 motifs monomères dérivant éventuellement de monomères choisis dans le groupe consistant en : des oxydes d'alcoylène, des alcoylène glycols, des épihalohydrines, des acides carboxyliques insaturés, des alcoylène imines, des lactones, de l'alcool vinylique et des alcanoates de vinyle.

7. Un cathéter intermittent emballé tel que revendiqué dans n'importe quelle revendication précédente, dans lequel le polymère de base comprend un polymère choisi dans le groupe consistant en : des polyoléfines, des polyesters, des polyacrylates, des polyamides, du matériau élastomère thermoplastique, un amide à séquence polyéther, des vulcanisats thermoplastiques, des copolyesters thermoplastiques, des polyamides thermoplastiques, du fluorocaoutchouc et des matières désintégrables par l'eau ou hydrolysables enzymatiquement ou leurs combinaisons, mélanges ou copolymères de l'un quelconque des matériaux ci-dessus.

8. Un cathéter intermittent emballé tel que revendiqué dans la revendication 7, dans lequel le polymère de base comprend un polymère choisi dans le groupe consistant en : des polyoléfines, du poly(chlorure de vinyle), du polyuréthane, un copolymère styrène-butadiène (SBC), un copolymère styrène-éthylène-butylène-styrène (SEBS) et du matériau élastomère thermoplastique ou leurs combinaisons, mélanges ou copolymères de l'un quelconque des matériaux ci-dessus.

9. Un cathéter intermittent emballé tel que revendiqué dans n'importe quelle revendication précédente, dans lequel l'additif amphiphile est un copolymère séquencé A-B comprenant une séquence de chaîne hydrocarbonée de la formule CH₃CH₂ (CH₂CH₂)ₐ, dans laquelle "a" va de 5 à 25 et de préférence de 9 à 25 et une séquence B hydrophile, dans laquelle la séquence B est un oligomère hydrophile comprenant entre 2 et 10 motifs monomères dérivés de monomères choisis dans le groupe consistant en : des oxydes d'alcoylène, des alcoylène glycols, des épihalohydrines, des acides carboxyliques insaturés, des alcoylène imines, des lactones, de l'alcool vinylique et des alcanoates de vinyle.

10. Un procédé de réduction de la migration d'un additif d'une surface d'un cathéter intermittent, le cathéter intermittent comprenant un corps tubulaire creux en polymère comprenant un polymère de base et un additif lubrifiant amphiphile, le procédé comprenant les stades d'emballage du cathéter intermittent dans un récipient contenant une solution aqueuse, dans lequel au moins une partie du cathéter intermittent est recouverte de la solution aqueuse, et dans lequel la solution aqueuse comprend au moins un sel en une concentration d'au moins 5 % en poids/volume.

11. Un procédé de réduction de la migration tel que revendiqué dans la revendication 10, dans lequel la solution aqueuse comprend au moins un agent tensio-actif et/ou un composé en poly(oxyde d'alcoylène) en une concentration totale d'au moins 0,005 mmol/L.

12. Utilisation d'une solution aqueuse comprenant au moins un sel en une concentration d'au moins 5 % en poids/volume ; pour réduire la migration d'un additif lubrifiant d'une surface d'un cathéter intermittent, le cathéter intermittent comprenant un corps tubulaire creux en polymère comprenant un polymère de base et un additif lubrifiant amphiphile.
